# EUROPEAN PATENT SPECIFICATION

(11) **EP 1 794 189 B1**
(45) Date of publication and mention of the grant of the patent: **04.06.2008**
(21) Application number: 05774775.0
(22) Date of filing: 29.08.2005
(51) Int. Cl.: C07K 14/47, C12P 21/06

(54) **ACE-INHIBITORY WHEY HYDROLYSATES**
ACE-HEMMENDE MOLKEHYDROLYSATE
HYDROLYSATS DU LACTOSERUM INHIBITEURS DE L'ENZYME DE CONVERSION DE L'ANGIOTENSINE (ECA)

(30) Priority: 31.08.2004 NL 1026931
(43) Date of publication of application: 13.06.2007
(73) Proprietor: Friesland Brands B.V., 7943 PE Meppel (NL)
(72) Inventor: DE SLEGTE, Jaap, NL-7006 KK Doetinchem (NL); KLARENBEEK, Gijsbertus, NL-7731 ST Ommen (NL)
(74) Representative: van Loon, C.J.J.
(86) International application number: PCT/NL2005/000622
(87) International publication number: WO 2006/025731

(56) References cited:
- WO-A-01/85984
- WO-A-03/063788
- NAKAMURA Y ET AL: "PURIFICATION AND CHARACTERIZATION OF ANGIOTENSIN I-CONVERTING ENZYME INHIBITORS FROM SOUR MILK" JOURNAL OF DAIRY SCIENCE, AMERICAN DAIRY SCIENCE ASSOCIATION. CHAMPAIGN, ILLINOIS, US, vol. 78, no. 4, 1995, pages 777-783, XP002912924 ISSN: 0022-0302 cited in the application
- ABUBAKAR A ET AL: "STRUCTURAL ANALYSIS OF NEW ANTIHYPERTENSIVE PEPTIDES DERIVED FROM CHEESE WHEY PROTEIN BY PROTEINASE K DIGESTION" JOURNAL OF DAIRY SCIENCE, AMERICAN DAIRY SCIENCE ASSOCIATION. CHAMPAIGN, ILLINOIS, US, vol. 81, no. 12, 1998, pages 3131-3138, XP002937027 ISSN: 0022-0302 cited in the application
- MULLALLY M M ET AL: "IDENTIFICATION OF A NOVEL ANGIOTENSIN-I-CONVERTING ENZYME INHIBITORY PEPTIDE CORRESPONDING TO A TRYPTIC FRAGMENT OF BOVINE BETA-LACTOGLOBULIN" FEBS LETTERS, ELSEVIER SCIENCE PUBLISHERS, AMSTERDAM, NL, vol. 402, no. 2/3, 3 February 1997 (1997-02-03), pages 99-101, XP001076810 ISSN: 0014-5793 cited in the application

## Description

The invention relates to compounds with an antihypertensive effect. More in particular, the invention relates to releasing ACE (angiotensin I-converting enzyme)-inhibitory peptides from whey proteins.

Hypertension or high blood pressure is an important risk factor for the development of heart and vascular diseases, which are the main cause of death in the Western world. It is estimated that 1 in 5 adults in the world suffer from hypertension. We refer to hypertension if the systolic blood pressure is on average 140 mm Hg or higher, and/or if the diastolic blood pressure is on average 90 mm Hg or higher. In general, lower values apply to pregnant women. Children have a blood pressure which is normally below 120/70 mm Hg. Hypertension accelerates the ageing of the arteries and increases the load on the heart. The older an artery, the greater the chance that it is damaged. Hypertension damages all arteries of the organism, which can lead to a heart attack, a stroke or damage of the kidneys. In order to avoid this, hypertension needs to be treated with medication. Conventionally, hypertension is prevented by administering antihypertensive medicines and by adjusting the dietary habits and lifestyle. ACE plays an important role in maintaining the blood pressure. In the human body, ACE brings about the formation of angiotensin II from angiotensin I. Increase of the amount of angiotensin II in the blood has *inter alia* the following effects: (1) constriction of the blood vessels (vasoconstriction) and associated hypertension; (2) release of aldosterone from the adrenal cortex and vasopressin (antidiuretic hormone = ADH) from the neurohypophysis; these two hormones cause the kidneys to retain more water, so that the blood volume increases; and (3) increase of the feeling of thirst, so that more water is drunk.

Blocking of ACE and prevention of an increase of the amount of angiotensin II in the blood result in a decrease of blood pressure in hypertensive patients. Therefore ACE inhibitors are important in the control of hypertension in medicine. However, a drawback of the many pharmaceutical preparations with synthetic ACE inhibitors which are on the market is that they often have unpleasant side effects, such as a dry cough.

In the literature, in the past 10 years, bioactive peptides potentially incorporated in milk proteins have regularly been written about. All kinds of hydrolysates have been manufactured from milk proteins, either with the aid of enzymes or *in situ* through fermentation processes (cheese, yoghurt), in which these bioactive peptides have been demonstrated. One of the most important properties of these bioactive peptides is blood pressure decrease through the inhibition of the ACE reaction. ACE-inhibitory peptides - either as protein hydrolysate or formed in situ through fermentation - are attractive 'natural' antihypertensive compounds.

Particular peptides are found to be very effective in the prevention and the treatment of hypertension. It is not exactly known what structural characteristics ACE-inhibitory peptides should satisfy, but there is some similarity between the peptides which already have a proven ACE inhibition. These are generally short peptides (2-4 amino acids) with a hydrophobic amino acid on the C-terminal side. Also, the amino acid proline is often mentioned which is then terminally, usually C-terminally present on the peptide.

The Japanese firm Calpis Co. Ltd has obtained ACE-inhibitory peptides through fermentation. These are usually small peptides which are characterized by the presence of the amino acid proline and a high ACE-inhibitory activity. The active peptides obtained by fermentation mainly consist of the sequences which have proline in their structures; namely Val-Pro-Pro or Ile-Pro-Pro (Nakamura, Y. et al. (1996), J. Dairy Sci. 78; 777-783). Other peptides with an antihypertensive activity are described in *inter alia* Nouchikusangiyou, Snow Brand, Symbicom AB and Nisshin Flour Milling Co Ltd [ref 10-17].

A number of companies have recently marketed hydrolysates and/or fermented products with an ACE-inhibitory activity, in combination with another property. Thus, WO 01/85984 (Davisco) describes a method for releasing ACE-inhibitory peptides from whey proteins with the aid of different types of industrially obtainable proteases, including porcine trypsin. Here, incidentally, not a protein concentrate was taken as a starting material, but a purified whey protein product, whey protein isolates. One hydrolysate, obtained through action of trypsin, was moreover found to have an antihypertensive effect after oral administration to rats. This hydrolysate has a degree of hydrolysis (%DH) of about 6% and has been marketed within the product range BIOZATE (www.daviscofoods.com). The degree of hydrolysis indicates to what extent peptide bonds of a protein have been hydrolyzed as a result of an enzymatic hydrolysis reaction. A %DH of 50 indicates that a particular enzyme has hydrolyzed 50% of the peptide bonds available for that enzyme. In addition, an additional property for such a type of hydrolysate has been described (WO 03/063778), where, in addition to ACE inhibition, the peptide mixture is also said to have a cholesterol (low-density lipoprotein) lowering effect.

WO 01/32906 of Valio describes the fermentation of casein with the aid of lactic acid bacteria to release peptides having an antihypertensive effect. The product produced by this means has been marketed as a fermented product under the name Evolus. This product is also said to have an additional property, namely increasing or enhancing the absorption of minerals under the influence of these peptides. These peptides are also characterized by high concentration of Val-Pro-Pro or Ile-Pro-Pro peptides, similar to the product of CALPIS.

It is obvious that it is important for a measurable blood pressure decrease that the active peptides / hydrolysates have sufficient ACE-inhibitory activity. The ACE-inhibitory activity of an ACE-inhibitor, such as a peptide or hydrolysate, is usually expressed as an IC50 value. This value indicates the concentration of peptide / hydrolysate necessary to reduce the ACE activity by 50%. The lower the IC50 value of an ACE inhibitor, the stronger the ACE-inhibitory activity.

The object of the invention is to provide hydrolysates with a strong ACE-inhibitory activity.

To this end, the invention provides a method for enzymatically producing a protein hydrolysate with angiotensin-converting enzyme (ACE)-inhibitory activity comprising treatment of a beta-lactoglobulin (hereinafter: BLG)-enriched protein with a broad-spectrum endoprotease in a first reaction step followed by treatment with a proline-specific protease in a second reaction step. Such a method yields a protein hydrolysate with an IC50 value which is at least three times lower compared with the known hydrolysates with ACE-inhibitory activity (see Table 1). In the first reaction step, the protein substrate is hydrolyzed to a fairly high degree of hydrolysis, usually about 10 to about 15%, in order to make proline-containing peptides available as a substrate for a proline-specific protease, so that, in the second step, peptides with a C-terminal proline-residue are formed.

A two-step hydrolysis process according to the invention with a first hydrolysis step with a broad-spectrum protease and a second hydrolysis step with a proline-specific protease applied to a BLG-rich protein substrate has not been described before. Though the use of BLG or a BLG-rich whey protein concentrate as a source of ACE-inhibitory peptides is known, in this, a proline-specific protease has not been used before. Abubakar et al. determined the ACE-inhibitory activity of whey protein hydrolysates prepared with 7 different enzymes: trypsin, proteinase-K, actinase-E, thermolysin, papain, pepsin and chymotrypsin. The enzyme specificity was found to have a great effect on the ACE-inhibitory activity of the resulting hydrolysates, and the biological activity was found to come from the 4 dominant proteins in whey (BLG; alpha-lactoglobulin, ALA; blood serum albumin, BSA; and immunoglobulins, Ig) (Abubakar et al. 1996, Tokohu J. Agric. Res., 47(1-2):1-8). More recent work of Abubakar et al. describes the identification of 9 peptide sequences, of which BLG tripeptide (f78-80; Ile-Pro-Ala) exhibited the strongest antihypertensive activity in hypertensive rats (Abubakar et al. 1998, J. Dairy Sc., 12:3131-3138). Mullally et al. isolated the peptide (f142-148) from a hydrolysate of bovine BLG obtained with trypsin. This peptide has an IC50 value of 43 µmol/L (Mullally et al. 1997. FEBS Letters, 402:99-101). Also, hydrolysates of BLG-enriched whey protein isolate have been prepared and tested for their ACE-inhibitory activity. WO 01/85984 (Davisco International Foods, Inc) describes the manufacture of ACE-inhibitory hydrolysates by means of porcine trypsin or a bacterial protease applied to different commercial whey protein concentrates. Here, a comparison is made between the ACE-inhibitory activity of a hydrolysate obtained from a standard whey protein isolate (94% protein) obtained by ion exchange chromatography (*Bi*PRO, Davisco Foods International, LeSueur, MN) and a BLG-enriched whey protein isolate. The hydrolysates obtained from the purified standard isolate were found to have a lower IC50 value (290-450 µg/ml) than the hydrolysates obtained from the BLG-enriched isolate (530-900 µg/ml). This was surprising, since peptide (f142-148) from BLG, which was used as a reference in the study, had the lowest IC50 value (40 µg/ml). So, just like the invention, WO01/85984 describes the manufacture of an ACE-inhibitory hydrolysate from a BLG-enriched whey protein concentrate. However, in WO01/85984, the manufacture involves one single hydrolysis step (either with trypsin, or with bacterial protease) whereas a method according to the present invention is characterized by a two-step hydrolysis process with a broad-spectrum protease followed by a proline-specific protease. In addition, the ACE-inhibitory activity of a hydrolysate according to the invention proves to be much higher than those of known (hydrolysates of) whey protein concentrates (see Table 3 below).

The present finding that hydrolysis of BLG with a proline-specific endoprotease (PSE) in a method according to the invention yields a hydrolysate with a relatively low IC50 value is very surprising. As stated hereinabove, particular powerful ACE-inhibitory tripeptides (e.g. Val-Pro-Pro or Ile-Pro-Pro) contain a high concentration of (terminal) proline residues. However, none of the known ACE-inhibitory peptides coming from BLG (see Table 2) contains a C-terminal proline residue, and only a few peptides (f(32-40); f(78-80); f(142-146) and f(142-148)) contain an internal proline residue. In addition, the total protein content in BLG is relatively low (5%) compared with, for instance, casein (11%), a much used milk protein for the formation of ACE-inhibitory peptides by means of (lactic acid) fermentation. Thus, under action of a PSE, relatively few peptides with a terminal proline residue are created. It is therefore not obvious to use a proline-specific endoprotease to release ACE-inhibitory peptides from BLG. The invention now shows that, after the two-step treatment according to the invention, a BLG-based hydrolysate has a higher ACE-inhibitory activity than a casein hydrolysate, in spite of the fact that casein contains a higher content of proline (see Table 1). One could conclude from this that particular specific amino acid sequences with a terminal proline are determinative of the ACE-inhibitory activity, and not terminal proline-containing peptides in general.

Preferably, a method according to the invention is used on a substrate consisting of a mixture of proteins of which at least 25% is BLG, such as 30% or 35% BLG, preferably at least 40% BLG, such as 45%, 50% or 55%, more preferably at least 60% BLG, such as 65% or 70%. Also, (purified) BLG can be used as a substrate. However, it goes without saying that the production costs of an ACE-inhibitory hydrolysate will be higher as a more purified protein substrate is used as a starting material.

A whey protein concentrate enriched with beta-lactoglobulin is an attractive starting material in a method according to the invention. A BLG-enriched WPC may be obtained by making the other whey proteins, mainly ALA, BSA and Ig, precipitate from a WPC by applying specific conditions. A particular combination of desalting, temperature and pH denatures the respective proteins, after which they aggregate and can be separated centrifugally or through membrane filtration.

Whey constitutes 80 to 90% of the total volume of milk entering the production process and contains about 50% of the nutrients of the original milk such as proteins, lactose, vitamins and minerals. In the past, whey has always been considered waste of the cheese industry, but the discharge of cheese whey causes large environmental problems. However, because the proteins have good functional properties, nowadays the interest in the use of whey for the extraction thereof is great. Only 0.55% of the whey consists of proteins. On a dry matter basis, this is 10%. The whey proteins are a heterogeneous mixture, of which the main proteins are B-lactoglobulin (50%), α-lactoglobulin (ALA; 20%), blood serum albumin (BSA; 5%) and immunoglobulins (Ig; 12%). In order to be able to increase the dry matter content of whey to at least approximately 50-60%, whey is concentrated or dried. A whey protein concentrate (WPC) is made by fractioning and concentrating whey by means of ultrafiltration. As further concentrating takes place, different levels of protein arise in the whey protein concentrate. Industrially produced WPCs are classified into the categories low-protein WPC (protein content between 25-40%), medium-protein WPC (protein content between 45-60%) and high-protein WPC (protein content between 60-80%). The higher the protein content of a WPC, the more BLG is available in the concentrate for hydrolysis to ACE-inhibitory peptides. Since whey proteins consist for approximately 50% of BLG, a high-protein WPC contains between 30-40% BLG, which is sufficient for use of such a WPC in a method according to the invention. However, the BLG content of low-protein and medium-protein WPCs is often too low. For WPCs with a protein content of about 55-60% or lower, it is therefore necessary to enrich for BLG. Suitable commercial WPCs include Domovictus 535 from BDI (35% protein of which 85% BLG); Hiprotal 880 from BDI (80% protein of which 50-55% BLG) and Hiprotal 580 (80% protein of which 80-85% BLG), all available from Borculo Domo Ingredients (BDI), Zwolle, the Netherlands.

As a broad-spectrum endoprotease in the first reaction step in a method according to the invention, different types of endoproteases can be used. Suitable endoproteases are of animal, vegetable or microbial origin. A microbial broad-spectrum endoprotease is preferred, since these are often easily commercially available at a relatively reasonable price. Chemically or genetically modified mutant proteases may be suitable as well. The broad-spectrum protease may be a serine protease or a metalloprotease, preferably an alkaline microbial protease. Examples of alkaline proteases are subtilisins (EC 3.4.21.62), in particular those coming from *Bacillus* spp., e.g. subtilisin Novo, subtilisin Carlsberg, subtilisin 309, subtilisin 147 and subtilisin 168 (described in WO 89/06279) and chymotrypsin (EC 3.4.21.1), which preferably hydrolyze the peptide bond on the C-terminal side of hydrophobic amino acids such as Tyr, Trp, Phe and Leu. Also, in the first reaction step, a mixture of two or more endoproteases can be used. Suitable commercially available proteases comprise Alcalase^{™}, Savinase^{™}, Primase^{™}, Everlase^{™}, Esperase^{™} and Kannase^{™} (Novozymes A/S), Maxatase^{™}, Maxacal^{™,} Maxapem^{™}, Properase^{™}, Purafect^{™}, Purafect OXP^{™}, FN2^{™}, and FN3^{™} (Genencor International Inc.), Corolase^{™} and Veron^{™} (AB Enzymes). Preferably, in the first reaction step, the enzyme Alcalase^{™} is used. In a further embodiment of the invention, as a broad-spectrum protease, an aspartate protease is used. Just like PSE, this enzyme has an acid pH optimum, which has the advantage that the pH does not need to be adjusted after the first step. Optionally, the aspartate protease is used simultaneously with the PSE in a one-step hydrolysis. However, in that case, the fact should be taken into account that the broad-spectrum protease can also hydrolyze the PSE enzyme. The duration of the first reaction step depends on various factors, such as the enzyme and/or the substrate used. Preferably, hydrolyzing takes place until a degree of hydrolysis of at least 8% has been reached, more preferably at least 10%, such as 12% or 15%. For most of the broad-spectrum proteases mentioned, it holds true that, under the conditions suitable for that enzyme (pH, temperature, salt concentration and the like), such a degree of hydrolysis can be reached in a few hours.

After the first incubation step, the broad-spectrum endoprotease used is preferably inactivated to prevent the possibility of the PSE being hydrolyzed by the broad-spectrum protease in the second reaction step. Methods for enzyme inactivation are known to a skilled person, such as a heat treatment or use of a compound which can inhibit the enzyme activity.

The second reaction step is carried out with a proline-specific endopeptidase (PSE; EC 3.4.21.26). A suitable PSE for use in the method of the invention is, for instance, a proline-specific endoprotease of *A. niger,* such as the enzyme which is known in the market under the name EndoPro and which is described in WO 02/45524. In a further embodiment, in the second reaction step, a Proline-Specific Endoprotease of *Flavobacterium meningosepticum* is used.

Further, the invention provides a protein hydrolysate with angiotensin-converting enzyme (ACE)-inhibitory activity, obtainable with a method as described hereinabove. This hydrolysate is characterized by a strong ACE-inhibitory activity (see Table 3) and can therefore be used with advantage for inhibiting ACE activity in mammals, such as humans. A hydrolysate is also suitable for veterinary application, for instance in a cat or dog. Since inhibition of ACE has an antihypertensive effect *in vivo,* an ACE-inhibitory hydrolysate can be used for the treatment of hypertension. A hydrolysate according to the invention can be processed into different products, such as liquid and dry products. If desired, downstream processing takes place, for instance in the form of an ultrafiltration (UF) treatment. The enzymes have a much larger molecular weight than the peptides and can be separated from the ACE-inhibitory peptides by means of UF. An additional advantage of a UF step is that the protein substrates which have not been degraded by the enzymes can also be separated from the biologically active hydrolysate.

It is assumed that, after oral intake, the peptides of a hydrolysate according to the invention end up in the stomach in intact or virtually intact form, to subsequently be taken up into the bloodstream. The broad-spectrum protease in the first reaction step of a method according to the invention has a broader substrate specificity than the brush border proteases in the intestinal wall, so that the hydrolysate according to the invention will not or hardly be hydrolyzed further after oral intake.

Optionally, a hydrolysate can be used in combination with other ACE inhibitors. In a further aspect of the invention, the ACE-inhibitory hydrolysate is preventively used to prevent hypertension, for instance in a person with a genetic disposition for hypertension or if medicines are used which cause hypertension as a side effect.

In addition to being effective with hypertension, ACE inhibitors also prove to be effective with heart failure. An ACE-inhibitory hydrolysate obtainable with a method according to the invention can therefore also be used with heart problems, for instance after a coronary to prevent recurrence.

Thus, the invention provides a treatment method for inhibiting ACE activity in a mammal, comprising the, preferably oral, administration of an ACE-inhibitory hydrolysate obtained according to the method of the invention in an amount and with a frequency the ACE activity.

Still another aspect of the present invention relates to a treatment method to reduce symptoms of hypertension in a mammal, comprising the, preferably oral, administration of a hydrolysate according to the invention in an amount and with a frequency which is sufficient to inhibit the symptoms of hypertension.

The invention also provides a composition comprising an ACE-inhibitory hydrolysate according to the invention. Such a composition with an ACE-inhibitory / antihypertensive activity is, for instance, a pharmaceutical composition or a food or luxury food. An ACE-inhibitory composition may have a solid or a liquid form. It is, for instance, a powder or a tablet. Preferably, the ACE-inhibitory composition according to the invention has a liquid form, such as a liquid milk product, a fruit juice or another type of drinkable product which can *inter alia* be used to prevent or treat hypertension.

Finally, the invention relates to the use of a composition according to the invention for the preparation of a medicine for (prophylactically) treating or preventing heart and vascular diseases. In particular, the medicine to be prepared is a medicine for inhibiting ACE activity in a mammal; a medicine for lowering the blood pressure; and/or a (prophylactic) medicine for the occurrence of hypertension.

### EXPERIMENTAL PART

### 1. In vitro measurement of ACE inhibition

The *in vitro* measurement of the ACE inhibitory activity of peptides or another product (e.g. hydrolysate) was carried out as follows:
- Product is solved in measuring buffer consisting of borate, sodium chloride at pH 8.3
- The substrate used for the measurement is Hippuryl-His-Leu from Sigma (H1635)
- The ACE enzyme also comes from Sigma (A6778)

In a microcuvette, the substrate is incubated at 38°C with ACE enzyme and activity is spectrophotometrically monitored at 260 nm. In the presence of a peptide or hydrolysate to be tested, the inhibition of this activity is measured and expressed as the required amount of product needed to reduce the activity of the enzyme by 50%. This inhibition is determined for four different concentrations of the product to be measured.

### Experiment 1

By means of a two-step hydrolysis process, ACE-inhibitory peptides are released from milk and whey proteins. In the first step, proteins were hydrolyzed with Alcalase (Novozymes), a broad-spectrum endoprotease of *Bacillus lichenformis.* To this end, 2.5 g of protein was solved in 50 ml water. The pH was set at 8.0 with 4 M NaOH. The reaction mixture was placed in a shaking water bath at a temperature of 60°C, after which the Alcalase (125µl pure enzyme) was added. The incubation was carried out for 4 hours.
At the end of the incubation period, the pH was brought to 5.0 with 4 M HCl. Then the broad-spectrum endoprotease was inactivated by an incubation of 5 minutes at 95°C. After this, the reaction mixture was ready for the second reaction step with the proline-specific endoprotease (PSE; also endoprolyl hydrolase) EndoPro (DSM), a prolyl hydrolase of *Aspergillus niger.*
In order to obtain an enzyme/substrate ratio of 1U per gram of protein, 556µl EndoPro was added to the reaction mixture. Then incubation took place at 50°C in a shaking water bath. At times t=0, 60, 180, 360 en 1440 minutes, 200-µl samples were taken which were ten times diluted and inactivated for 5 minutes at 95°C. Then the IC50 was determined of the hydrolysates obtained. The results are shown in Table 1. The protein substrates tested in this experiment are:
1. Domovictus 535 (BDI); a WPC with approximately 35% protein of which approximately 85% BLG
2. Hiprotal 580 (BDI); a WPC with 80% protein of which 80% BLG
3. Casein (Sigma)
4. Hiprotal 880 (BDI); a WPC with 80% protein of which 50% BLG
5. Beta-lactoglobulin (Sigma)
6. Alpha-lactalbumin (Sigma)

**Table 1. Effect of treatment with proline-specific protease on ACE-inhibitory activity expressed as IC50 (µg/ml) of a protein substrate which was priorly treated with a broad-spectrum protease.**

| Nr | Protein substrate | t=0 (min) | 60 | 180 | 360 | 1440 |
|---|---|---|---|---|---|---|
| 1 | Domovictus 535 | 30.1 | 23.1 | 14.6 | 15.4 | 16.8 |
| 2 | Hiprotal 1580 | 20.1 | 13.8 | 7.6 | 11.2 | 14.7 |
| 3 | Casein | 64.2 | 35.2 | 27.2 | 23.2 | 19.8 |
| 4 | Hiprotal 880 | 20.4 | 14.8 | 11.4 | 10.1 | 20.3 |
| 5 | β-lactoglobulin | 23.0 | 14.6 | 13.3 | 10.8 | 18.7 |
| 6 | α-lactalbumin | 26.9 | 24.5 | 26.9 | 24.0 | 20.8 |

After the first hydrolysis step with a broad-spectrum protease, the hydrolysates have an IC50 value varying from 20.1 µg/ml (Hiprotal 580) to 64.2 µg/ml (casein) as shown in column t=0 of Table 1. A follow-up treatment with a proline-specific endoprotease (PSE) for 60 minutes proves to be sufficient for Hiprotal 580, Hiprotal 880 and BLG to obtain an IC50 value below 15 µg/ml. A 3-hour incubation with PSE results in a further decrease of the IC50 values, while, for the Hiprotal 580 hydrolysate (also referred to as Hydrohiprotal 980) a very low value of 7.6 µg/ml is obtained. Prolongation of the incubation period to 6 hours (t=360 min) with a proline-specific protease yields a further (small) decrease of the IC50 value for a number of protein substrates, while, for most substrates, the IC50 values show an increase again after a 24-hour incubation (t=1440 min). So, there appears to be an optimal incubation period, which could be explained by the fact that, with advanced hydrolysis, the peptides with ACE-inhibitory activity just formed are degraded further to peptides with a lower ACE-inhibitory activity.

The results in Table 1 show that hydrolysates prepared from a BLG-rich protein substrate (Domovictus 535, Hiprotal 580, Hiprotal 880 and BLG) according to a two-step procedure have an angiotensin-I-converting enzyme (ACE)-inhibitory activity which is about three times lower than has been observed so far for whey proteins. It further appears from the results of Table 1 that, in spite of a higher content of proline, the casein substrate (11% proline) yields a lower ACE-inhibitory activity after the two-step enzyme treatment than the hydrolysate on the basis of BLG-rich substrate (5% proline). Apparently, particular specific sequences with terminal proline are determinative of the ACE-inhibitory activity, and not only terminal proline-containing peptides.

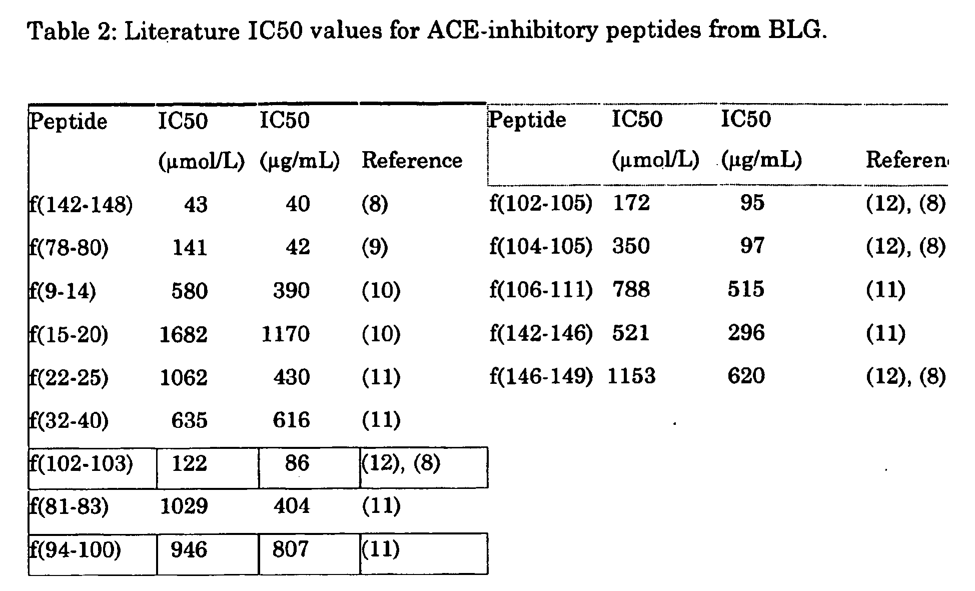

### Experiment 2

In this experiment, the *in vitro* ACE-inhibitory activity of a hydrolysate according to the invention (Hiprotal 580 treated for 20 hours with Alcalase followed by 3 hours of hydrolysis with proline-specific endoprotease) was compared with the ACE-inhibitory activity of the following commercially available hydrolysates, some of which have an antihypertensive effect.
- Hydrolysate DMV (standard protein hydrolysate for use in children's food)
- CE90 ACE (DMV product which is alleged to inhibit ACE; see www.dmv-international.com)
- Hydrolysate DI 3065 (Arla) (79.8% protein)
- Hydrolysate LE 80 BM (DMV) (81.7% protein)
- Hydrolysate CE 90 STL (DMV) (83.5% protein)
- Hydrolysate CE90 CPP (DMV) (87.0% protein)

The amount of protein present is between approximately 80 and 90% for the different products. Prior to the measurement of the ACE-inhibitory activity, 200-250 mg of product was dissolved in 20 ml of incubation buffer. After the product had dissolved completely, the solution was diluted 40x with incubation buffer. This dilution was used for determining the ACE-inhibitory activity. The results are shown in Table 3.

**Table 3: Experimental IC50 values of commercial protein hydrolysates compared with an ACE-inhibitory hydrolysate according to the invention.**

| Product | IC50 |
|---|---|
| | µg/ml |
| Hydrolysate of the invention | 7.2 |
| Hydrolysate DMV | 47.0 |
| CE90 ACE (DMV) | 35.0 |
| Hydrolysate DI 3065 (Arla) | 33.0 |
| Hydrolysate LE 80 BM (DMV) | 44 |
| Hydrolysate CE90 STL (DMV) | >44 |
| Hydrolysate CE90 CPP (DMV) | >44 |
| Evolus (VALIO) | >44 |
| Biozate (DAVISCO) | 62 |

It appears from the results obtained that the IC50 values of some commercial products (DMV, Arla, Davisco) are in the same range as those of standard milk protein hydrolysates (30-40µg/ml). Surprisingly, the IC50 value of the BLG-enriched hydrolysate according to the invention is about 5 times lower (approximately 7 µg/ml) and accordingly the activity is 5 times higher than other standard whey protein hydrolysates.

### REFERENCES

1. Process for producing fermented milk containing angiotensin converting enzyme inhibitory peptide and process for producing milk serum; CALPIS CO., LTD.; EP 1142481A1
2. Process for producing fermented milk containing angiotensin converting enzyme inhibitory peptide and process for producing milk serum; CALPIS CO., LTD.; EP 1142481A4
3. HIGH YIELD PRODUCTION OF CURDS AND WHEY CONTAINING AN ACE INHIBITOR PEPTIDE (VAL-PRO-PRO AND/OR ILE-PRO-PRO) MADE UNDER STIRRING CONDITIONS; CALPIS CO., LTD; NZ 0513305A
4. Process for producing granules containing angiotensin-converting enzyme inhibiting peptides; CALPIS CO., LTD.; US 6428812
5. GRANULES FOR ORAL ADMINISTRATION, PROCESS FOR PRODUCING THE SAME, AND TABLETS PRODUCED FROM THE GRANULES; CALPIS CO., LTD.; WO 0041677A1
6. GRANULATED MATERIAL, ITS PRODUCTION AND TABLET USING THE SAME; CALPIS CO., LTD.; JP 200020405A2
7. Method of purifying whey of lactic acid fermentation by electrodialysis; CALPIS CO. LTD; US 6204362
8. Mullally et al.; Identification of a novel angiotensin-I-converting enzyme inhibitory peptide corresponding to a tryptic fragment of bovine β-lactoglobulin; FEBS Lett. 402(1997), 99-101.
9. Abubakar et al.; Structural analysis of new antihypertensive peptides derived from cheese whey protein by proteinase K digestion; J. of Dairy Sci. 81(1998), 3131-3139.
10. Pihlanto-Leppala et al.; Angiotensin-I-converting enzyme inhibitory peptides derived from bovine milk proteins; Int. Dairy J. 8(1998), 325-331.
11. Pihlanto-Leppala et al.; Angiotensin-I-converting enzyme inhibitory properties of whey protein digests: concentration and characterization of active peptides; J. of Dairy Res. 67(2000), 53-64.
12. NEW PEPTIDE AND ITS UTILIZATION; SNOW BRAND CO. LTD.; JP 0829088A2
13. Human β-casein, process for producing it and use thereof; SYMBICOM AKTIEBOLAG; US 5739407
14. GENE ENCODING A HUMAN BETA-CASEIN PROCESS FOR OBTAINING THE PROTEIN AND USE THEREOF IN AN INFANT FORMULA; SYMBICOM EP 0599978A1
15. GENE ENCODING A HUMAN BETA-CASEIN PROCESS FOR OBTAINING THE PROTEIN AND USE THEREOF IN AN INFANT FORMULA; SYMBICOM AKTIEBOLAG; WO 9304172A3
16. GENE ENCODING A HUMAN BETA-CASEIN PROCESS FOR OBTAINING THE PROTEIN AND USE THEREOF IN AN INFANT FORMULA; SYMBICOM AKTIEBOLAG; WO 9304172A2
17. New peptide and angiotensin transferase inhibitor; NISSHIN FLOUR MILLING CO. LTD; JP 04082898A2
18. Enzymatic treatment of whey proteins for the production of antihypertensive peptides, the resulting products and treatment of hypertension in mammals; DAVISCO; WO 0185984
19. Reducing cholesterol with hydrolyzed whey protein; DAVISCO; WO 03/063778 A2.
20. Process for producing a product containing antihypertensive tripeptides; VALIO; WO 01/32906.

## Claims

1. A method for enzymatically producing a protein hydrolysate with angiotensin-converting enzyme (ACE)-inhibitory activity, **characterized by** treatment of a beta-lactoglobulin (BLG)-containing substrate with a broad-spectrum endoprotease in a first reaction step followed by treatment with a proline-specific endoprotease in second reaction step.

2. A method according to claim 1, **characterized in that** the substrate is a whey protein concentrate (WPC), preferably a WPC which has been enriched with beta-lactoglobulin (BLG).

3. A method according to claim 1 or 2, **characterized in that** the substrate contains at least 25%, preferably at least 35%, more preferably at least 60% BLG.

4. A method according to any one of claims 1-3, **characterized in that** the broad-spectrum endoprotease is a serine protease, a metalloprotease or an aspartate protease.

5. A method according to any one of claims 1-4, **characterized in that** the broad-spectrum endoprotease is a microbial protease, preferably a protease isolated from *Bacillus lichenformis* or *Bacillus subtilis.*

6. A method according to any one of claims 1-5, **characterized in that** the proline-specific endoprotease is a proline-specific endoprotease according to EC 3.4.21.26.

7. A method according to any one of claims 1-6, wherein the first reaction step is carried out until a degree of hydrolysis of about 10 to about 15% has been reached.

8. A method according to any one of claims 1-7, wherein the second reaction step is carried out for at least 0.5 hour, preferably at least 1 hour, more preferably at least 2 hours, most preferably at least 3 hours.

9. A method according to any one of claims 1-8, wherein the second reaction step is carried out for at most 24 hours, preferably at most 16 hours, more preferably at most 10 hours.

10. A protein hydrolysate with angiotensin-converting enzyme (ACE)-inhibitory activity, obtainable with a method according to any one of claims 1-9.

11. A composition comprising a hydrolysate according to claim 10, preferably a pharmaceutical composition or a food or luxury food.

12. A composition according to claim 11, wherein the composition is a liquid product, such as a milk product or a fruit juice, or a solid product, such as a powder.

13. Use of a composition according to claim 10 or 11 for the preparation of a medicine for inhibiting ACE activity in a mammal, preferably a human, for lowering the blood pressure; and/or for preventing the occurrence of hypertension.

14. Use according to claim 13 wherein the mammal is a human with hypertension or an increased risk of hypertension.

15. Use according to claim 13 or 14 wherein the inhibiting of ACE activity comprises the oral administration of a composition according to claim 11 or 12 in an amount and with a frequency sufficient to inhibit the ACE activity.

16. Use according to claim 13 or 14 wherein the reduction of hypertension comprises the oral administration of a composition according to claim 10 or 11 in an amount and with a frequency sufficient to inhibit the symptoms of hypertension.

## Patentansprüche

1. Verfahren zur enzymatischen Erzeugung eines Proteinhydrolysats mit Angiotensin-umwandelnder Enzym-(ACE)-inhibitorischer Aktivität, **gekennzeichnet durch** eine Behandlung eines Beta-Lactoglobulin-(BLG)-haltigen Substrats mit einer Breitspektrum-Endoprotease in einem ersten Reaktionsschritt, gefolgt von einer Behandlung mit einer Prolin-spezifischen Endoprotease in einem zweiten Reaktionsschritt.

2. Verfahren gemäß Anspruch 1, **dadurch gekennzeichnet, daß** das Substrat ein Molkeproteinkonzentrat (WPC) ist, vorzugsweise ein WPV, das mit Beta-Lactoglobulin (BLG) angereichert wurde.

3. Verfahren gemäß Anspruch 1 oder 2, **dadurch gekennzeichnet, daß** das Substrat mindestens 25 %, vorzugsweise mindestens 35 %, noch bevorzugter mindestens 60 % BLG enthält.

4. Verfahren gemäß einem der Ansprüche 1 bis 3, **dadurch gekennzeichnet, daß** die Breitspektrum-Endoprotease eine Serinprotease, eine Metalloprotease oder eine Aspartatprotease ist.

5. Verfahren gemäß einem der Ansprüche 1 bis 4, **dadurch gekennzeichnet, daß** die Breitspektrum-Endoprotease eine mikrobielle Protease, vorzugsweise eine Protease, die von Bacillus lichenformis oder Bacillus subtilis isoliert wurde, ist.

6. Verfahren gemäß einem der Ansprüche 1 bis 5, **dadurch gekennzeichnet, daß** die Prolin-spezifische Endoprotease eine Prolin-spezifische Endoprotease gemäß EC 3.4.21.26 ist.

7. Verfahren gemäß einem der Ansprüche 1 bis 6, wobei der erste Reaktionsschritt durchgeführt wird, bis ein Hydrolysegrad von ungefähr 10 bis ungefähr 15 % erreicht wurde.

8. Verfahren gemäß einem der Ansprüche 1 bis 7, wobei der zweite Reaktionsschritt für mindestens 0,5 Stunden, vorzugsweise mindestens 1 Stunde, noch bevorzugter mindestens 2 Stunden, besonders bevorzugt mindestens 3 Stunden durchgeführt wird.

9. Verfahren gemäß einem der Ansprüche 1 bis 8, wobei der zweite Reaktionsschritt für höchstens 24 Stunden, vorzugsweise höchstens 16 Stunden, noch bevorzugter höchstens 10 Stunden durchgeführt wird.

10. Proteinhydrolysat mit Angiotensin-umwandelnder Enzym-(ACE)-inhibitorischer Aktivität, erhältlich durch ein Verfahren gemäß einem der Ansprüche 1 bis 9.

11. Zusammensetzung, umfassend ein Hydrolysat gemäß Anspruch 10, vorzugsweise eine pharmazeutische Zusammensetzung oder ein Nahrungsmittel oder Luxusnahrungsmittel.

12. Zusammensetzung gemäß Anspruch 11, wobei die Zusammensetzung ein Flüssigprodukt wie ein Milchprodukt oder ein Fruchtsaft oder ein festes Produkt wie in Pulver ist.

13. Verwendung einer Zusammensetzung gemäß Anspruch 10 oder 11 für die Herstellung einer Medizin zur Inhibition der ACE-Aktivität bei einem Säuger, vorzugsweise einem Menschen, zur Erniedrigung des Blutdrucks und/oder zur Verhinderung des Auftretens eines Bluthochdrucks.

14. Verwendung gemäß Anspruch 13, wobei der Säuger ein Mensch mit einem Bluthochdruck oder einem erhöhten Risiko für einen Bluthochdruck ist.

15. Verwendung gemäß Anspruch 13 oder 14, wobei die Inhibition der ACE-Aktivität die orale Verabreichung einer Zusammensetzung gemäß Anspruch 11 oder 12 in einer ausreichenden Menge und mit einer ausreichenden Frequenz umfaßt, um die ACE-Aktivität zu inhibieren.

16. Verwendung gemäß Anspruch 13 oder 14, wobei die Reduktion des Bluthochdrucks die orale Verabreichung einer Zusammensetzung gemäß Anspruch 10 oder 11 in einer ausreichenden Menge und mit einer ausreichenden Frequenz zur Inhibition der Symptome des Bluthochdrucks umfaßt.

## Revendications

1. Procédé pour produire enzymatiquement un hydrolysat de protéines avec une activité inhibitrice d'enzyme de conversion de l'angiotensine (ECA), **caractérisé par** le traitement d'un substrat contenant de la bêta-lactoglobuline (BLG) par une endoprotéase à large spectre dans une première étape de réaction, suivi d'un traitement par une endoprotéase spécifique de la proline dans une seconde étape de réaction.

2. Procédé selon la revendication 1, **caractérisé en ce que** le substrat est un concentré de protéines de lactosérum (WPC, de préférence un WPC qui a été enrichi en bêta-lactoglobuline (BLG).

3. Procédé selon la revendication 1 ou 2, **caractérisé en ce que** le substrat contient au moins 25 %, de préférence 35 %, mieux encore au moins 60 % de BLG.

4. Procédé selon l'une quelconque des revendications 1-3, **caractérisé en ce que** l'endoprotéase à large spectre est une sérine protéase, une métalloprotéase ou un aspartate protéase.

5. Procédé selon l'une quelconque des revendications 1-4, **caractérisé en ce que** l'endoprotéase à large spectre est une protéase microbienne, de préférence une protéase isolée à partir de *Bacillus lichenformis* ou *Bacillus subtilis.*

6. Procédé selon l'une quelconque des revendications 1-5, **caractérisé en ce que** l'endoprotéase spécifique de la proline est une endoprotéase spécifique de la proline conformément à EC 3.4.21.26.

7. Procédé selon l'une quelconque des revendications 1-6, dans lequel la première étape de réaction est mise en oeuvre jusqu'à atteindre un degré d'hydrolyse d'environ 10 à environ 15 %.

8. Procédé selon l'une quelconque des revendications 1-7, dans lequel la deuxième étape de réaction est mise en oeuvre pendant au moins 0,5 heure, de préférence au moins 1 heure, mieux encore au moins 2 heures, mieux encore au moins 3 heures.

9. Procédé selon l'une quelconque des revendications 1-8, dans lequel la deuxième étape de réaction est mise en oeuvre pendant au plus 24 heures, de préférence au plus 16 heures, mieux encore au plus 10 heures.

10. Hydrolysat de protéines avec une activité inhibitrice d'enzyme de conversion de l'angiotensine (ECA), pouvant être obtenu par un procédé selon l'une quelconque des revendications 1-9.

11. Composition comprenant un hydrolysat selon la revendication 10, de préférence une composition pharmaceutique ou un aliment ou un aliment de luxe.

12. Composition selon la revendication 11, la composition étant un produit liquide, tel qu'un produit laitier ou un jus de fruit, ou un produit solide, tel qu'une poudre.

13. Utilisation d'une composition selon la revendication 10 ou 11 pour la préparation d'un médicament pour inhiber une activité ACE chez un mammifère, de préférence un humain, pour réduire la pression sanguine ; et/ou pour prévenir l'apparition de l'hypertension.

14. Utilisation selon la revendication 13, dans laquelle le mammifère est un humain présentant une hypertension ou un risque accru d'hypertension.

15. Utilisation selon la revendication 13 ou 14, dans laquelle l'inhibition de l'activité ACE comprend l'administration orale d'une composition selon la revendication 11 ou 12 en une quantité et avec une fréquence suffisantes pour inhiber l'activité ACE.

16. Utilisation selon la revendication 13 ou 14, dans laquelle la réduction de l'hypertension comprend l'administration orale d'une composition selon la revendication 10 ou 11 en une quantité et avec une fréquence suffisantes pour inhiber les symptômes de l'hypertension.
